# EUROPÄISCHE PATENTANMELDUNG

(11) **EP 1 072 636 A1**
(43) Veröffentlichungstag der Anmeldung: **31.01.2001**
(21) Anmeldenummer: 00116035.7
(22) Anmeldetag: 26.07.2000
(51) Int. Cl.: C08K 3/00, C08K 5/00, C08K 3/26, C08L 5/04

(54) **Vernetzung ionotroper Gele**

(30) Priorität: 28.07.1999 DE 19935231
(71) Anmelder: Zimmermann, Ulrich, 97295 Waldbrunn (DE)
(72) Erfinder: Zimmermann, Ulrich, 97295 Waldbrunn (DE)
(74) Vertreter: Draudt, Jutta, Dr.

(57) **Zusammenfassung**

Ein Vernetzungsmittel zur Vernetzung ionotroper Gele durch Verbindung von Gelmolekülen über Gegenionenbrücken enthält die Gegenionen in einem an eine Trägersubstanz gebundenen Zustand. Die Gegenionen sind durch eine äußere Stoff-, Temperatur- oder Strahlungseinwirkung von der Trägersubstanz lösbar. Zur Gelvernetzung wird zunächst ein Gemisch aus den zu vernetzenden Gelmolekülen und dem Vernetzungsmittel bereitgestellt, aus diesem Gemisch ein Formkörper gebildet und dann durch eine äußere Stoff-, Temperatur- oder Strahlungseinwirkung die Vernetzung der Gelmoleküle durch Freigabe der Gegenionen von der Trägersubstanz ausgelöst.

## Beschreibung

Die Erfindung betrifft ein Vernetzungsmittel zur Vernetzung ionotroper Gele, Stoffzusammensetzungen aus ionotropen Gelen und derartigen Vernetzungsmitteln, ein Verfahren zur Vernetzung ionotroper Gele und Anwendungen des Vernetzungsmittels.

Ionotrope Gele sind hochelastische, reversibel quellbare und ionenaustauschende Gele, die aus Makromolekülen bestehen, welche durch Ionenbrücken miteinander vernetzt sind. Die Ionenbrücken werden durch Gegenionen zu den Gelmolekülen gebildet. Es ist bekannt, wässrig gelöste ionotrope Gele einfach dadurch zu vernetzen, daß der Gellösung eine geeignete Gegenionenlösung zugesetzt wird. Diese Vernetzung im Lösungsvolumen besitzt jedoch den Nachteil, daß die Vernetzung oder Polymerisation ungleichförmig verläuft, da gerade in den Volumenbereichen, in denen die Vernetzung bereits stattgefunden hat, der Stofftransport von Gegenionen zu weiteren vernetzbaren Gelmolekülen behindert wird. Im Ergebnis der herkömmlichen Vernetzung in der Lösung ergeben sich somit auf molekularer Ebene ungleichmäßig vernetzte Gele, die zu Endprodukten mit unregelmäßigen Geloberflächen führen. Diese Inhomogenität stellt einen Nachteil dar, der die Anwendung vernetzter Gele, insbesondere im Bereich der Biologie und Medizin, stark einschränkt.

Ein Beispiel für ionotrope Gele ist durch die aus Algen gewonnenen Alginate gegeben. Alginate besitzen zahlreiche Anwendungen in der Lebensmitteltechnik (s. z.B. Askar in "Alimenta", Bd. 21, 1982, S. 165 ff.) und auch in der Medizin, Biochemie und Biotechnologie. Beispielsweise wird in DE-OS 42 04 012 die Bildung von mit Ba²⁺-Ionen vernetzten Alginatkapseln für Anwendungen in der Implantationsmedizin beschrieben. Die Alginatkapseln werden hergestellt, indem eine Alginatlösung mit den einzukapselnden biologischen Zellen tropfenweise in eine Ba²⁺-Lösung gegeben wird. Beim Eintropfen in die Ionenlösung beginnt unmittelbar die Gelvernetzung, die zu geschlossenen Alginatkapseln führt. Allerdings tritt auch bei dieser Kapselbildung das obengenannte Problem einer inhomogenen Vernetzung auf. Die Vernetzung beginnt von der äußeren Oberfläche her, die selbst nach Vernetzung die Ionenbrückenbildung zwischen Gelmolekülen unterhalb der Oberfläche behindert. Im Ergebnis besitzen die Alginatkapseln in molekularem Maßstab unregelmäßige Oberflächen.

Untersuchungen mit dem Rasterkraftmikroskop (AFM) haben ergeben, daß eine mögliche immunologische Reaktivität der Oberfläche von Alginatkapseln gerade durch geometrische Inhomogenitäten hervorgerufen werden können. Die zellulären Prozesse, die die primären Fremdkörperreaktionen hervorrufen (d.h. die Absonderung von Materialspuren durch Fibroplasten, Makrophagen und/oder Lymphozyten während ihrer Wanderung über Oberflächen), werden durch Vernetzungsinhomogenitäten nachteilig beeinflußt.

Neben der Beeinflussung der immunologischen Reaktivität von Alginattransplantaten beschränkt die Vernetzungsinhomogenität ferner in nachteiliger Weise die Langzeitstabilität der transplantierten Kapseln. Die Alginatkapseln sind im transplantierten Zustand je nach den Umgebungsverhältnissen einer Wasseraufnahme oder einem Wasserentzug ausgesetzt, so daß eine Kapselverformung und damit einhergehend eine Kraftwirkung auf das Kapselmaterial auftritt. Es ist davon auszugehen, daß die inhomogene Vernetzung von Alginaten die Widerstandsfähigkeit der Kapseln gegenüber derartigen Kraftwirkungen nachteilig verringert.

Die genannten Probleme treten auch bei anderen ionotropen Gelen auf, die beispielsweise in der Zahnmedizin eingesetzt werden. Es besteht daher ein Interesse, ionotrope Gele selbst auf molekularer Ebene homogen zu vernetzen, um die bisherigen Beschränkungen bei den Anwendungen in der Biologie und Medizin zu überwinden.

Die Aufgabe der Erfindung ist es, ein verbessertes Vernetzungsmittel für ionotrope Gele anzugeben, mit dem eine homogene Gelvernetzung erzielt wird. Die Aufgabe der Erfindung ist es auch, ein verbessertes Vernetzungsverfahren zur Bildung homogen vernetzter Gele und neuartige Anwendungen der Gele zu beschreiben. Diese Aufgaben werden durch ein Vernetzungsmittel, eine Gellösung, eine Pulverzusammensetzung und ein Verfahren mit den Merkmalen entsprechend den Patentansprüchen 1, 7, 8 bzw. 9 gelöst. Vorteilhafte Ausführungsformen und Anwendungen der Erfindung ergeben sich aus den abhängigen Ansprüchen.

Die Grundidee der Erfindung besteht in der Schaffung eines Vernetzungsmittels zur Vernetzung ionotroper Gele durch Verbindung von Gelmolekülen über Gegenionen-Brücken, bei dem das Vernetzungsmittel die Gegenionen in einem an eine Trägersubstanz gebundenen Zustand enthält, wobei die Gegenionen durch eine äußere Stoff-, Temperatur- oder Strahlungseinwirkung von der Trägersubstanz lösbar sind. Ein derartiges Vernetzungsmittel ermöglicht die Durchführung eines neuartigen Vernetzungsverfahrens, bei dem zunächst eine homogene Durchmischung der zu vernetzenden Gelmoleküle einerseits und der an die Trägersubstanz gebundenen Gegenionen andererseits bereitgestellt und in eine anwendungsabhängig gewünschte Form gebracht wird, woraufhin durch Auslösung der Stoff-, Temperatur- oder Strahlungseinwirkung die Gegenionen im Vernetzungsmittel freigesetzt werden und die Gegenionen Brücken zwischen den Gelmolekülen aufbauen. Diese Durchmischung der Ausgangskomponenten kann entweder im gelösten Zustand oder auch im getrockneten, fein pulverisierten Zustand gegeben sein. Im Unterschied zur herkömmlichen Vernetzung, bei der die Gegenionenbrückenbildung unmittelbar beim Vermengen der gelösten Ausgangskomponenten mit den oben ausgeführten Nachteilen für die Homogenität der Vernetzung gestartet wird, kann erfindungsgemäß zunächst für eine gleichmäßige Verteilung des Vernetzungsmittels zwischen den ionotropen Molekülen gesorgt werden. In den Ausgangskomponenten sind vor der Vernetzung die Gegenionen im noch unwirksamen, da an die Trägersubstanz gebundenen Zustand räumlich homogen verteilt, so daß bei einsetzender Vernetzung nach Beginn der äußeren Stoff-, Temperatur- oder Strahlungseinwirkung keine nachteilige Behinderung des Gegenionentransports auftritt.

Bei bevorzugten Ausführungsformen des erfindungsgemäßen Vernetzungsmittels wird die Trägersubstanz durch sogenannte Käfigsubstanzen oder Cage-Substanzen gebildet, die in einem elektronischen Grundzustand die Gegenionen (multivalente Kationen oder Anionen) binden und in einem elektronischen Anregungszustand die Gegenionen frei geben. Anwendungsabhängig werden bevorzugt Cage-Substanzen für divalente Kationen, z.B. Ca²⁺- oder Ba²⁺-Cageverbindungen, verwendet. Der Übergang von dem einen zum anderen Zustand kann vorteilhafterweise durch eine einfache Bestrahlung mit Licht einer geeigneten Wellenlänge ausgelöst werden. Gemäß einer abgewandelten Ausführungsform der Erfindung sind die Gegenionen und die Trägersubstanz als Salzverbindung im Vernetzungsmittel enthalten, die durch eine Änderung des pH-Wertes der Umgebungslösung auflösbar ist.

Die Erfindung liefert die folgenden Vorteile. Die erfindungsgemäße Vernetzung ermöglicht eine im gesamten Ausgangsvolumen simultan einsetzende und damit homogen verlaufende Gelvernetzung. Damit können Gelproben mit einer auf molekularer Ebene homogenen und glatten Oberfläche geschaffen werden, die bei Anwendung als Transplantate oder als Füllstoffe in der Zahnmedizin eine verbesserte Biokompatibilität bzw. Stabilität zeigen. Mit der Erfindung werden aber auch völlig neue Anwendungen ionotroper Gele geschaffen, da mit dem neuen Vernetzungsmittel die Handhabung der Gele wesentlich vereinfacht wird.

Weitere Einzelheiten und Vorteile der Erfindung werden aus der Beschreibung der beigefügten Zeichnungen ersichtlich. Es zeigen:
- Fig. 1: eine schematische Illustration der Wirkung des erfindungsgemäßen Vernetzungsmittels,
- Fig. 2: eine schematische Darstellung einer ersten Anordnung zur Implementierung des erfindungsgemäßen Verfahrens,
- Fig. 3: eine schematische Darstellung einer weiteren Anordnung zur Implementierung des erfindungsgemäßen Verfahrens,
- Fig. 4: eine Illustration zur Anwendung der Erfindung bei der Bildung von Wundverbänden, und
- Fig. 5: eine Illustration zur Anwendung der Erfindung in der Zahnmedizin.

Die Erfindung wird im folgenden unter Bezug auf die Vernetzung von Alginaten mit zweiwertigen Kationen beschrieben. Der Bezug auf Alginate wird hier nur beispielhaft gegeben. Die Erfindung ist in entsprechender Weise auch bei anderen ionotropen Gelen, z.B. bei DEAE-Polyhydrodyverbindungen, insbesondere für kosmetische Anwendungen, und bei Polysacchariden anwendbar, die unter dem Namen Sephadex (registrierte Marke) bekannt sind. Weitere Beispiele sind ionotrope Zellulosederivate und Anionen- oder Kationenaustauscher wie DEAA-Zellulose, DEAE-Zellulose, ECTEOLA-Zellulose, TEAE-Zellulose, DEAE-Sephadex, n-Octylamino-Sephadex, Polyaminopolystyren, Amberlite IR-45, Amberlite IRA-93, Amberlite IRA-410, oder Amberlite IRA-900, bzw. CM-Zellulose, Zellulose-Citrat, P-Zellulose, Amberlite CG-50, Amberlite IRC-50, Amberlite IR-120, Amberlite IR-200, Amberlite XE-97, oder Dowex-50. Die im folgenden erläuterten Prinzipien der Erfindung sind dabei entsprechend anwendbar.

Ein erfindungsgemäßes Vernetzungsmittel besteht aus einer Trägersubstanz-Gegenionen-Verbindung. Diese Verbindung kann physikalischer oder chemischer Natur sein. Bei der Verwendung von Cage-Substanzen als Trägersubstanz wird jeweils ein Gegenion von einem Cage-Molekül wie in einer käfigförmigen Umhüllung gehalten. Beispiele für Cage-Substanzen sind z.B. DM-Nitrophen (registrierte Marke von Calbiochem Novabiochem), welches zur Käfigbildung für Ca²⁺- oder Mg²⁺-Ionen geeignet ist, oder die von J. H. Kaplan et al. in "Proc. Nat. Acad. Sci. USA", Bd. 85, 1988, S. 6571 ff. beschriebenen Chelatmoleküle, die ebenfalls als Cagesubstanzen für Ca²⁺-Ionen geeignet sind. Die Cage-Substanzen zeichnen sich dadurch aus, daß sie im Grundzustand die käfigförmige Molekülgestalt besitzen und bei elektronischer Anregung einer Konformationsänderung eingehen, so daß das gefangene Ion freigegeben wird. Diese elektronische Anregung erfolgt beispielsweise durch eine UV-Belichtung.

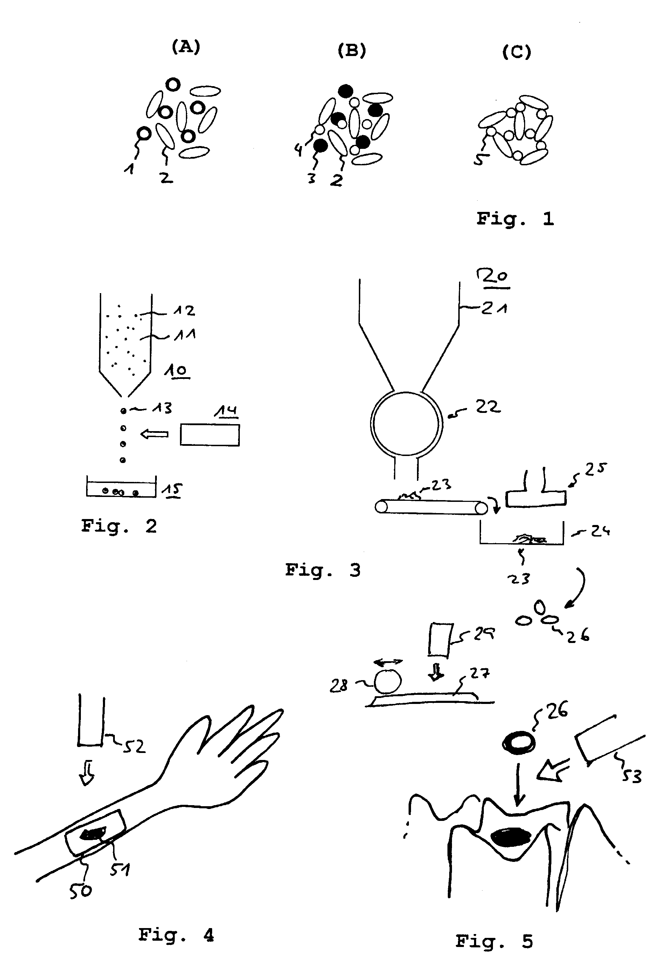

Fig. 1 illustriert die Schritte einer Gelvernetzung mit einem erfindungsgemäßen Vernetzungsmittel. Das linke Bild (A) zeigt eine homogene Vermischung des Vernetzungsmittels 1 und der Gelmoleküle 2. Die schematisch gezeigten Partikel sind nicht notwendigerweise Einzelmoleküle, sondern gegebenenfalls auch größere Molekülverbände, bis hin zu Pulverpartikeln. Die Zusammensetzung im Zustand (A) ist entweder trocken (Pulverzustand) oder gelöst (Lösungszustand, Umgebungslösungsmittel nicht eingezeichnet).

Bei UV-Belichtung geht das System in den Zustand (B) (mittleres Bild) über, in dem sich das Vernetzungsmittel in die Trägersubstanz 3 und die Gegenionen 4 aufgelöst hat. Die Gegenionen 4 sind damit im gesamten Volumen, in dem die zu vernetzenden Gelmoleküle verteilt sind, ebenfalls homogen verteilt. Unmittelbar nach der Freisetzung beginnt die Vernetzung (Bildung der Gegenionenbrücken 5), die zum Zustand (C) führt. Das vernetzte Gel zeichnet sich durch eine homogen verteilte Brückenbildung aus. Gegebenenfalls ist zusätzlich ein Schritt zur Extraktion der restlichen Trägersubstanz vorgesehen.

Alternativ zur Bildung der Trägersubstanz durch Cage-Substanzen kann das Vernetzungsmittel als Trägersubstanz auch Ionen enthalten, die mit den Gegenionen zur Gelvernetzung ein Salz bilden. Ein für die Alginatvernetzung durch Kalziumbrückenbildung interessierendes Vernetzungsmittel ist beispielsweise Kalziumcarbonat oder Bariumcarbonat. In diesem Fall wird die Freigabe der Gegenionen nicht durch eine Belichtung, sondern durch eine stoffliche Änderung im Umgebungslösungsmittel erzielt. Durch eine Ansäuerung der Lösung oder Suspension von Alginat und Kalziumcarbonat werden die Kalziumionen freigesetzt und damit der Vernetzung des Alginats zur Verfügung gestellt.

Bei weiteren Vernetzungsmitteln kann vorgesehen sein, daß die Trägersubstanz das Gegenion bei Erwärmung freigibt.

Fig. 2 illustriert eine erste Anwendung des erfindungsgemäßen Vernetzungsverfahrens. Zur Bildung von Alginatkapseln mit einer Düsenanordnung, wird in einem Tropfenerzeuger 10 eine Suspension aus einer Alginatlösung 11, der das Vernetzungsmittel gelöst zugesetzt ist, und biologischen Zellen 12, die verkapselt werden sollen, bereitgestellt. Mit einem anwendungsabhängig gewählten Mechanismus werden Suspensionstropfen 13, die jeweils in der Regel eine zu umhüllende Zelle enthalten, freigegeben. Unmittelbar nach Freigabe werden die Tropfen mit einer Belichtungseinrichtung 14 (z.B. Blitzlampe, UV-Laser oder dgl.) belichtet. Dies entspricht dem Übergang vom Zustand (A) zum Zustand (B) gemäß Fig. 1. Die Gegenionen im Vernetzungsmittel werden freigegeben und die Vernetzung beginnt. Die vernetzten Kapseln werden in einer Auffangeinrichtung 15 gesammelt und anschließend ihrer weiteren Verwendung, z.B. einer Transplantation in einem lebenden Organismus, zugeführt. Die im Tropfenerzeuger 10 bereitgestellte Alginatlösung 11 aus Gelmolekülen und Vernetzungsmittel stellt einen wichtigen Gesichtspunkt der Erfindung dar, da sie vorteilhafterweise bereits vor dem Zusatz der lebenden Zellen langzeitstabil produziert und anwendungsabhängig eingesetzt werden kann.

Ein alternatives, lösungsmittelfreies Verfahren ist schematisch in Fig. 3 illustriert. In einer Mühle 20 ist ein Vorratsbehälter 21 vorgesehen, in dem die getrockneten Ausgangskomponenten aus unvernetztem Gelmaterial und Vernetzungsmittel vorgemischt werden. In einem Mahlwerk 22 werden die Ausgangskomponenten gemahlen, so daß sich ein homogenes Gemisch entsprechend dem Zustand (A) in Fig. 1 ergibt. Dieses Gemisch 23 wird in einer Auffangeinrichtung 24 gesammelt und beispielsweise mit einer Preßeinrichtung 25 zu Tabletten oder Pellets 26 verpreßt. Diese Tabletten 26 werden dann der weiteren Anwendung (s. unten) zugeführt. Unmittelbar vor der gewünschten Vernetzung erfolgt ein Anlösen oder Aufquellen des verpreßten Pulvers und die Belichtung oder Temperierung oder Stoffzuführung zur Freigabe der Gegenionen von der Trägersubstanz im Vernetzugsmittel. Alternativ kann das Gemisch 23 auch als Schicht 27 mit einer Beschichtungseinrichtung 28 auf einem geeigneten Substrat aufgetragen werden. Nach einem geeigneten Vorquellen oder Anlösen erfolgt eine Belichtung der gequollenen oder angelösten Schicht 27 mit einer Belichtungseinrichtung zur Auslösung der Gelvernetzung. Anschließend kann die Gelschicht vom Substrat abgelöst, ggf. zerkleinert und der weiteren Anwendung zugeführt werden.

Im folgenden werden bevorzugte Anwendungen erfindungsgemäß vernetzter Gele beschrieben.

### Bildung von Wundverbänden

Fig. 4 illustriert eine neuartige und besonderes vorteilhafte Anwendung ionotroper Gele zur Bildung von provisorischen Wundverbänden, z.B. bei Arbeits- oder Sportverletzungen. Eine Gellösung, die eine wässrige Lösung der zu vernetzenden Gelmoleküle und des Vernetzungsmittels umfaßt, wird als Abdeckschicht 50 auf der Verletzung 51 aufgebracht. Während oder unmittelbar nach dem Aufbringen der Schicht 50 erfolgt mit einer Belichtungseinrichtung 52 die Freigabe der Gegenionen aus dem Vernetzungsmittel, so daß die Vernetzung des Gels gestartet und nach kurzer Zeit die geschlossene Gelschicht ausgebildet ist.

Bei dieser Anwendung wirkt sich die Biokompatibilität ionotroper Gele besonders vorteilhaft aus, da mit dem direkt aufgebrachten Wundverband lediglich Stoffe mit dem freiliegenden Gewebe in Kontakt kommen, die hautfreundlich sind und eine vernachlässigbare oder keine Abwehrreaktion auslösen.

Ein in der beschriebenen Art hergestellter Wundverband eignet sich besonders gut zum Schutz gegen Verbrennungen, da die stabil vernetzten Alginate einen zuverlässigen Austtrocknungsschutz für das abgedeckt Gewebe darstellen.

### Bildung provisorischer Zahnfüllungen

Eine weitere Anwendung der Erfindung, insbesondere der gemäß Fig. 3 hergestellten, verpreßten Pulverzusammensetzung 26 liegt in der Schaffung von Zahnfüllungen. Die Tablette 26 wird als provisorische Füllung in einen Hohlraum im Zahn eingeführt, angelöst oder aufgequollen und einer Belichtung mit der Belichtungseinrichtung 53 unterzogen. Unmittelbar durch die Belichtung wird die Freigabe der Gegenionen im gesamten Körper der Tablette 26 ausgelöst.

Analoge Anwendungen ergeben sich bei der Schaffung provisorischer Implante in Knochenmaterialien.

### Anwendungen in der Lebensmitteltechnik

Erfindungsgemäß vernetzte Alginate lassen sich mit besonderem Vorteil in an sich bekannter Weise als Bestandteil von Lebensmitteln zur Veränderung von deren Festigkeits- oder Geschmackseigenschaften verwenden.

Aufgrund der hohen Stabilität der erfindungsgemäß homogenen Gelvernetzung eignen sich die Alginate besonders zur geschmacksneutralen Verkapselung von Wirkstoffen in Lebensmitteln. Beispielsweise kann Fischöl in erfindungsgemäß vernetzten Alginaten eingekapselt und in Brot verbacken werden. Aufgrund der erhöhten Stabilität der homogenen Vernetzung bleiben die Alginatkapseln auch beim Backen und Verspeisen stabil, so daß eine vom Wirkstoff unbeeinflußte Geschmacksneutralität bewahrt bleibt.

Weitere analoge Anwendungen ergeben sich bei der Wirkstoffverkapselung in der Kosmetik.

## Patentansprüche

1. Vernetzungsmittel (1) zur Vernetzung ionotroper Gele durch Verbindung von Gelmolekülen (2) über Gegenionenbrücken (5), **dadurch gekennzeichnet**, daß das Vernetzungsmittel die Gegenionen (4) in einem an eine Trägersubstanz (3) gebundenen Zustand enthält und die Gegenionen (4) durch eine äußere Stoff-, Temperatur- oder Strahlungseinwirkung von der Trägersubstanz (3) lösbar sind.

2. Vernetzungsmittel gemäß Anspruch 1, bei dem die Trägersubstanz aus Käfigmolekülen besteht, die in einem elektronischen Grundzustand die Gegenionen binden und in einem elektronischen Anregungszustand die Gegenionen freigeben.

3. Vernertzungsmittel gemäß Anspruch 2, bei dem die Käfigmoleküle aus Cage-Substanzen gebildet sind, wie sie in der Zellphysiologie zum Transport divalenter Ionen in biologische Zellen verwendet werden.

4. Vernetzungsmittel gemäß Anspruch 1, bei dem die Trägersubstanz und das Gegenion eine Salzverbindung bilden, die durch Einwirkung einer ansäuernden Lösung lösbar ist.

5. Vernetzungsmittel gemäß Anspruch 4, das aus Kalziumcarbonat besteht.

6. Vernetzungsmittel gemäß einem der vorhergehenden Ansprüche, das zur Vernetzung von Alginsäuremolekülen ausgelegt ist.

7. Gellösung, die ein ionotropes Gel und ein Vernetzungsmittel gemäß einem der Ansprüche 1 bis 6 enthält.

8. Pulverzusammensetzung bestehend aus einem getrockneten, nicht-vernetzten ionotropen Gel und einem getrockneten Vernetzungsmittel gemäß einem der Ansprüche 1 bis 6.

9. Verfahren zur Vernetzung ionotroper Gele unter Verwendung eines Vernetzungsmittels gemäß einem der Ansprüche 1 bis 6, mit den Schritten:
- Bereitstellung eines Gemisches aus den zu vernetzenden Gelmolekülen und dem Vernetzungsmittel,
- Bildung eines Schicht- oder Volumenformkörpers aus dem Gemisch, und
- Vernetzen der Gelmoleküle durch eine äußere Stoff-, Temperatur- oder Strahlungseinwirkung, durch die die Gegenionen von der Trägersubstanz gelöst werden.

10. Verfahren gemäß Anspruch 1, bei dem der erste Schritt die Bereitstellung einer wässrigen Lösung der zu vernetzenden Gelmoleküle und den Zusatz des Vernetzungsmittels umfaßt.

11. Verfahren gemäß Anspruch 9, bei dem der erste Schritt ein Vermischen und Vermahlen von Pulver aus den nicht-vernetzten Gelmolekülen und dem Vernetzungsmittel umfaßt.

12. Verfahren gemäß einem der Ansprüche 9 bis 11, bei dem das Vernetzen durch eine UV-Belichtung ausgelöst wird.

13. Verfahren gemäß einem der Ansprüche 9 bis 11, bei dem das Vernetzen durch eine Ansäuerung ausgelöst wird.

14. Verfahren gemäß einem der Ansprüche 9 bis 13, bei dem das vernetzte ionotrope Gel in Kapselform gebildet wird.

15. Verfahren gemäß Anspruch 14, bei dem in den Kapseln lebende biologische Zellen eingehüllt sind.

16. Anwendung eines Vernetzungsmittels gemäß einem der Ansprüche 1 bis 6 oder einer Gellösung gemäß Anspruch 7 oder einer Pulverzusammensetzung gemäß Anspruch 8 zur:
- Bildung von Wundverbänden,
- Bildung von Zahnfüllungen,
- Bildung von Transplantatverkapselungen,
- Bildung von Wirkstoffverkapselungen für die Lebensmitteltechnik, und
- Bildung von Wirkstoffverkapselungen für die Kosmetik.
